# EUROPEAN PATENT APPLICATION

(11) **EP 4 570 845 A1**
(43) Date of publication of application: **18.06.2025**
(21) Application number: 23216132.3
(22) Date of filing: 13.12.2023
(51) Int. Cl.: C08G 61/12, C07D 519/00, C08L 65/00, C09D 165/00, C09K 11/06, H10K 30/00

(54) **NOVEL POLYTHIOPHENE/POLYANION COMPOSITIONS**

(71) Applicant: AGFA-GEVAERT NV, 2640 Mortsel (BE)
(72) Inventor: HERCKENS, Roald, 2640 Mortsel (BE); LOCCUFIER, Johan, 2640 Mortsel (BE)
(74) Representative: Viaene, Kris

(57) **Abstract**

A polythiophene/polyanion composition comprising:
i) an oligo- or polythiophene obtained by the polymerization of a monomer according to Formula I, and 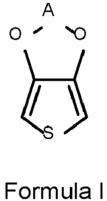 wherein
A represents a substituted or unsubstituted C1 to C5 alkylene bridge,
ii) a polyanion obtained by the polymerization of at least one monomer according to Formula II, 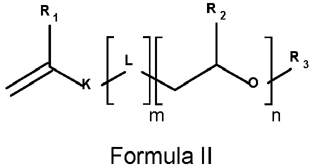 wherein
R₁ is selected from the group consisting of hydrogen, a substituted or unsubstituted alkyl group, a substituted or unsubstituted alkenyl group, a substituted or unsubstituted alkynyl group, a substituted or unsubstituted aralkyl group, a substituted or unsubstituted alkaryl group and a substituted or unsubstituted aryl or heteroaryl group; K is selected from the group consisting of a substituted or unsubstituted alkylene group, an ether group, an ester group, a substituted or unsubstituted amide group, a substituted or unsubstituted alkenylene group, a substituted or unsubstituted alkynylene group, a substituted or unsubstituted aralkylene group, a substituted or unsubstituted alkarylene group and a substituted or unsubstituted arylene or heteroarylene group;
L is selected from the group consisting of a substituted or unsubstituted alkylene group, a substituted or unsubstituted alkenylene group, an amine group, an ether group, a thioether group, a substituted or unsubstituted alkynylene group, a substituted or unsubstituted aralkylene group, a substituted or unsubstituted alkarylene group and a substituted or unsubstituted arylene or heteroarylene group;
R₂ and R₃ are independently selected from the group consisting of hydrogen and a substituted or unsubstituted alkyl group;
m represents 0 or 1;
n is an integer from 1 to 10 000;

and wherein the amount of monomer according to Formula II in the polyanion is from 0.1 to 20 mol %, relative to the total amount of monomers.

## Description

### Technical field of the Invention

The present invention relates to novel polythiophene/polyanion compositions and their use in polymer capacitors and Li-ion batteries.

### Background art for the invention

Environmental concerns about greenhouse gas emissions have stimulated the demand for electric and (plug in) hybrid electric vehicles (EVs).

The electrical systems inside these vehicles require improved battery and capacitor materials to improve properties such as battery capacity, cycling stability or charging speed.

Batteries used in EVs are typically Li-ion batteries (LIB). To improve the energy density of such LIBs, cathode materials with a higher intrinsic capacity are explored. Conventional LIBs have a Li Nickel Manganese Cobalt oxide (NMC) cathode in which equal amounts of the transition metals are used (referred to as NMC111). Increasing the amount of Nickel results in a higher intrinsic capacity of the cathode, however at the cost of a lower stability. These stability problems may be minimized by covering the cathode surface with a conductive polymer layer. For example, Poly(3,4-ethylenedioxythiophene) applied by chemical vapor deposition onto a Ni-rich NMC cathode improved the cycling stability of the battery (Liu et al.,ACS Nano 2022 16(9), 14527-14538). However, the development of industrially viable coating methods for applying the conductive polymer on the NMC cathode material is still lacking which limits the applicability of this concept.

Besides batteries, EVs also require improved capacitors, which are used in the control units of these vehicles. Hybrid polymer aluminum electrolytic capacitors are often applied in this field. These capacitors consist of an etched Al/Al₂O₃ foil, which serves as respectively the anode and the dielectric layer, covered by a conductive polymer layer, which functions as the cathode. Typically, Poly(3,4-ethylenedioxythiophene):poly(styrene sulfonate) (PEDOT:PSS) is used, a conductive polymer complex that is coated on the Al/Al₂O₃ substrate by dip-coating from an aqueous dispersion. To increase the surface area of the capacitor, an etched Al foil is used that is anodized to provide a thin layer of Al₂O₃. This process creates a porous Al/Al₂O₃ substrate, which is then dip-coated with the aqueous PEDOT:PSS dispersion. A sufficient penetration of PEDOT:PSS particles into the Al/Al₂O₃ substrate is necessary to obtain optimal capacitor properties. Smaller PEDOT:PSS particles for example resulted in improved capacitor properties, as disclosed in for example EP-A 1927120 (HERAEUS PRECIOUS METALS GMBH&CO).

In the present inventions, it has been observed that using an alternative polyanion instead of PSS results in improved properties of both LIBs and polymer capacitors. Alternative polyanions for PSS according to the present invention are copolymers of styrene- or acrylate- based polyethylene glycols and aromatic sulfonic acids. It has been observed that using such complexes between PEDOT and these novel polyanions results in a lower equivalent series resistance (ESR) and an improved capacitance of Polymer capacitors and improved capacity and cycling performance of Li-ion batteries. In addition, aqueous dispersions of these novel polythiophene/polyanion complexes may be applied by an industrially feasible wet-coating method.

EP-A 3459127 (KEMET ELECTRONICS CORP) discloses the use of copolymers of styrene sulfonic acid (PSS) and comonomers containing a polyethylene glycol group (PEO) as polyanion for a conductive polymer and their use in capacitors. The comonomer can be acrylate- and styrene-based. The molecular weight of the poly(ethylene glycol) group is up to 200 000 Da and a ratio (wt/wt) of the PSS/PEO monomers is between 0.01 and 100, for example 50/50 and 80/20.

Kayser et al. (Chem.Mater. 2018, 30, 13, 4459-4468) disclose sodium 4-styrene-sulfonate and poly(ethylene glycol methylether acrylate) blockcopolymers. The amount of ethylene glycol monomer is 70 mol %.

Tsutsumi et al. disclose the use of sulfonic acid copolymers with acrylic poly(ethylene glycol)s in 50/50 to 70/30 ratio as a dopant for polyaniline. These composites were used as an electrode for lithium ion concentration batteries (Synthetic Metals 97 (1998) 53-56) and as electrode in a solid-state battery (J. Electrochem.Soc., Vol.142, N°.9, September 1995).

### Summary of the invention

It is an object of the invention to provide novel polythiophene/polyanion compositions with which polymer capacitors having improved equivalent series resistance (ESR) and capacitance and Li-ion batteries having improved capacity and cycle life may be obtained.

The object of the invention is realized by the polythiophene/polyanion composition as defined in claim 1.

Other objects of the invention are a polymer capacitor and a Li-ion battery both comprising the polythiophene/polyanion composition and the preparation thereof.

Further objects of the invention will become apparent from the description hereinafter.

### Detailed description of the invention

### Definitions

The term "monofunctional" in e.g. monofunctional polymerizable compound means that the polymerizable compound includes one polymerizable group.

The term "difunctional" in e.g. difunctional polymerizable compound means that the polymerizable compound includes two polymerizable groups.

The term "polyfunctional" in e.g. polyfunctional polymerizable compound means that the polymerizable compound includes more than two polymerizable groups.

The term "alkyl" means all variants possible for each number of carbon atoms in the alkyl group i.e. methyl, ethyl, for three carbon atoms: n-propyl and isopropyl; for four carbon atoms: n-butyl, isobutyl and tertiary-butyl; for five carbon atoms: n-pentyl, 1,1-dimethyl-propyl, 2,2-dimethylpropyl and 2-methyl-butyl, etc.

Unless otherwise specified a substituted or unsubstituted alkyl group is preferably a C₁ to C₆-alkyl group.

Unless otherwise specified a substituted or unsubstituted alkenyl group is preferably a C₂ to C₆-alkenyl group.

Unless otherwise specified a substituted or unsubstituted alkynyl group is preferably a C₂ to C₆-alkynyl group.

Unless otherwise specified a substituted or unsubstituted alkaryl group is preferably a phenyl or naphthyl group including one, two, three or more C₁ to C₆-alkyl groups.

Unless otherwise specified a substituted or unsubstituted aralkyl group is preferably a C₇ to C₂₀-alkyl group including a phenyl group or naphthyl group.

Unless otherwise specified a substituted or unsubstituted aryl group is preferably a phenyl group or naphthyl group.

Unless otherwise specified a substituted or unsubstituted heteroaryl group is preferably a five- or six-membered ring substituted by one, two or three oxygen atoms, nitrogen atoms, sulphur atoms, selenium atoms or combinations thereof.

Unless otherwise specified a substituted or unsubstituted alkylene group is preferably a C₁ to C₆-alkylene group.

The term "substituted", in e.g. substituted alkyl group means that the alkyl group may be substituted by other atoms than the atoms normally present in such a group, i.e. carbon and hydrogen. For example, a substituted alkyl group may include a halogen atom or a thiol group. An unsubstituted alkyl group contains only carbon and hydrogen atoms.

Unless otherwise specified a substituted alkyl group, a substituted alkenyl group, a substituted alkynyl group, a substituted aralkyl group, a substituted alkaryl group, a substituted aryl and a substituted heteroaryl group are preferably substituted by one or more constituents selected from the group consisting of methyl, ethyl, n-propyl, isopropyl, n-butyl, isobutyl and tertiary-butyl, ester, amide, amine, ether, thioether, ketone, aldehyde, sulfoxide, sulfone, sulfonate ester, sulphonamide, -Cl, -Br, -I, -OH, -SH, -CN and -NO₂.

### Polythiophene/polyanion composition

The polythiophene/polyanion composition according to the present invention comprises:
i) an oligo- or polythiophene obtained by the polymerization of at least one monomer according to Formula I, and 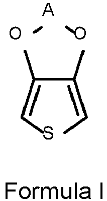 wherein
   A represents a substituted or unsubstituted C₁ to C₅ alkylene bridge,
ii) a polyanion obtained by the polymerization of at least one monomer according to Formula II, 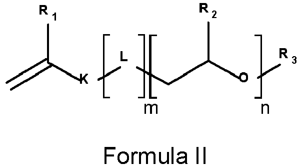 wherein
   R₁ is selected from the group consisting of hydrogen, a substituted or unsubstituted alkyl group, a substituted or unsubstituted alkenyl group, a substituted or unsubstituted alkynyl group, a substituted or unsubstituted aralkyl group, a substituted or unsubstituted alkaryl group and a substituted or unsubstituted aryl or heteroaryl group;
   K is selected from the group consisting of a substituted or unsubstituted alkylene group, an ether group, an ester group , a substituted or unsubstituted amide group, a substituted or unsubstituted alkenylene group, a substituted or unsubstituted alkynylene group, a substituted or unsubstituted aralkylene group, a substituted or unsubstituted alkarylene group and a substituted or unsubstituted arylene or heteroarylene group;
   L is selected from the group consisting of a substituted or unsubstituted alkylene group, a substituted or unsubstituted alkenylene group, an ether group, a thioether group, an amine group, a substituted or unsubstituted alkynylene group, a substituted or unsubstituted aralkylene group, a substituted or unsubstituted alkarylene group and a substituted or unsubstituted arylene or heteroarylene group;
   R₂ and R₃ are independently selected from the group consisting of hydrogen and a substituted or unsubstituted alkyl group;
   m represents 0 or 1;
   n is an integer from 1 to 10 000;
   wherein the total amount of monomers according to Formula II in the polyanion is from 0.1 to 20 mol % relative to the total amount of monomers.

The composition is preferably an aqueous dispersion comprising oligo- or polythiophene/polyanion particles. The dispersion medium is preferably water. However, the dispersion medium may also comprise water-soluble organic solvents such as alcohols or acids.

The median particle size (d₅₀) of the polythiophene/polyanion particles is preferably from 1 to 100 nm, more preferably from 2 to 75 nm, most preferably from 5 to 50 nm, particularly preferred from 10 to 40 nm. The d₅₀ particle size is preferably measured by centrifugal liquid sedimentation particle size analysis or by laser diffraction analysis.

The polythiophene/polyanion composition may comprise additional ingredients typically dependent on the application for which the composition is used. Such additional ingredients are for example selected from solvents, surface-active compounds, adhesion promoters, crosslinking agents, binders, conductivity increasing compounds, other conductive agents, heat and moisture stability improving agents, acidic compounds, alkaline compounds and electro-active materials.

The polythiophene/polyanion composition may have a pH from 1 to 14, more preferably from 1 to 8. The pH of the composition is typically optimized for the application wherein it will be used. When used in polymer capacitors, the pH is preferably from 4 to 9 to avoid corrosion of the dielectric. Also, when used for Li-ion batteries, the pH of the cathode active composition (see below) is preferably from 4 to 9.

The viscosity of the polythiophene/polyanion composition is typically optimized as function of the application method and may be from 1 to 1 000 mPa s, measured with a rheometer at 20 °C and a shear rate of 100 s⁻¹. Preferably, the viscosity is from 5 to 500 mPa s, more preferably from 10 to 250 mPa s.

The adjustment of the viscosity can, for example, be accomplished by adding appropriate rheology modifiers as a further additive. When used in polymer capacitors, the viscosity may not be too high to ensure a good application/ penetration of the dispersion on and in the porous dielectric. When used in Li-ion batteries, the viscosity may not be too low to ensure a proper coating of the cathode active composition onto the current collector.

### Oligo- or polythiophene

The polythiophene according to the present invention is an oligo- or polythiophene obtained by the polymerization of at least one monomer according to Formula I, 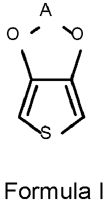 wherein
A represents a substituted or unsubstituted C₁ to C₅ alkylene bridge.

The term C₁ to C₅ alkylene bridge as used in Formula I means an alkylene bridge comprising 1 to 5 carbon atoms.

The C₁ to C₅ alkylene bridge is preferably methylene, ethylene, n-propylene, n-butylene or n-pentylene, most preferably ethylene.

In a particular preferred embodiment, the monomer according to Formula I is 3,4-ethylenedioxythiophene (EDOT).

The polythiophene may be a homopolymer or a copolymer.

The polythiophene is preferably poly(3,4-ethylenedioxythiophene) (PEDOT).

### Polyanion

The polyanion according to the present invention is obtained by the polymerization of at least one monomer according to Formula II, 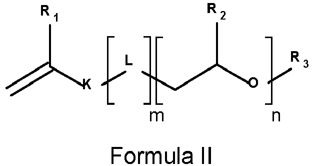 wherein
R₁ is selected from the group consisting of hydrogen, a substituted or unsubstituted alkyl group, a substituted or unsubstituted alkenyl group, a substituted or unsubstituted alkynyl group, a substituted or unsubstituted aralkyl group, a substituted or unsubstituted alkaryl group and a substituted or unsubstituted aryl or heteroaryl group;
K is selected from the group consisting of a substituted or unsubstituted alkylene group, an ether group , an ester group , a substituted or unsubstituted amide group, a substituted or unsubstituted alkenylene group, a substituted or unsubstituted alkynylene group, a substituted or unsubstituted aralkylene group, a substituted or unsubstituted alkarylene group and a substituted or unsubstituted arylene or heteroarylene group;
L is selected from the group consisting of a substituted or unsubstituted alkylene group, a substituted or unsubstituted alkenylene group, an ether group, a thioether group, an amine group, a substituted or unsubstituted alkynylene group, a substituted or unsubstituted aralkylene group, a substituted or unsubstituted alkarylene group and a substituted or unsubstituted arylene or heteroarylene group;
R₂ and R₃ are independently selected from the group consisting of hydrogen and a substituted or unsubstituted alkyl group;
m represents 0 or 1;
n is an integer from 1 to 10 000;
wherein the total amount of monomers according to Formula II in the polyanion is from 0.1 to 20 mol % relative to the total amount of monomers.

R₁ is preferably selected from the group consisting of hydrogen and a substituted or unsubstituted alkyl group, more preferably from the group consisting of hydrogen and a methyl group.

K is preferably selected from the group consisting of a substituted or unsubstituted arylene group, an ester group and an amide group, more preferably K represents a phenylene group or an ester group.

L is preferably selected from the group consisting of a substituted or unsubstituted alkylene group, an ether group and a thioether group, more preferably L represents an unsubstituted alkylene group.

R₂ and R₃ are preferably selected from the group consisting of hydrogen and an unsubstituted alkyl group. More preferably, R₂ represents a hydrogen and R₃ represents a methyl group.

Typical monomers according to Formula II are disclosed in Table 1 without being limited thereto.

**Table 1**

| | |
|---|---|
| 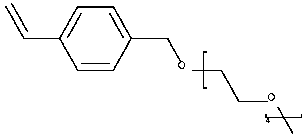 | Monomer-01 |
| 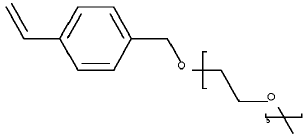 | Monomer-02 |
| 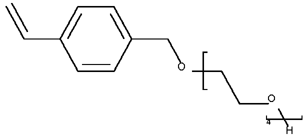 | Monomer-03 |
| 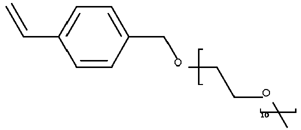 | Monomer-04 |
| 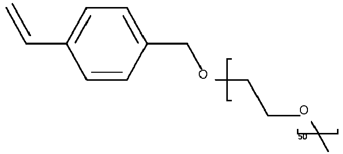 | Monomer-05 |
| 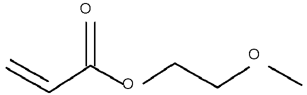 | Monomer-06 |
| 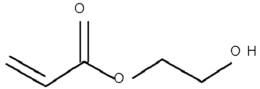 | Monomer-07 |
| 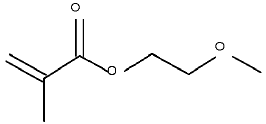 | Monomer-08 |
| 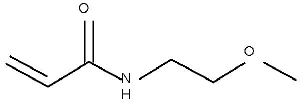 | Monomer-09 |
| 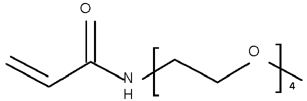 | Monomer-10 |
| 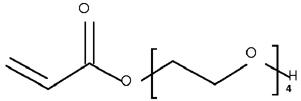 | Monomer-11 |
| 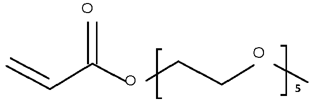 | Monomer-12 |
| 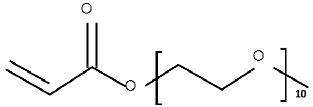 | Monomer-13 |
| 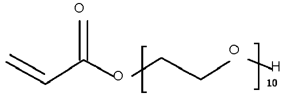 | Monomer-14 |
| 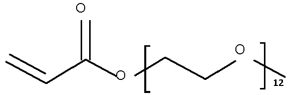 | Monomer-15 |
| 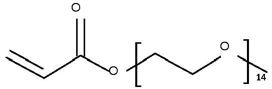 | Monomer-16 |
| 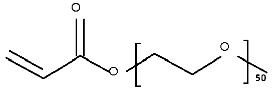 | Monomer-17 |
| 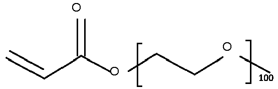 | Monomer-18 |
| 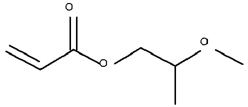 | Monomer-19 |
| 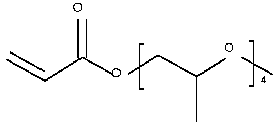 | Monomer-20 |

The polyanion is preferably a copolymer obtained by the polymerization of at least one monomer according to Formula II and at least one other monomer wherein the total amount of monomers according to Formula II is from 0.1 to 20 mol%, more preferably from 1 to 15 mol%, most preferably from 2 to 10 mol%, all relative to the total amount of monomers of the polyanion. Copolymers with styrene sulfonic acid are particularly preferred copolymers.

The weight average molecular weight (Mw) of the polymeric anion is preferably from 1 to 1000 kDa, more preferably from 50 to 500 kDa, most preferably from 100 to 300 kDa.

Typical polyanions according to the invention are shown in Table 2 without being limited thereto.

**Table 2**

| | |
|---|---|
| 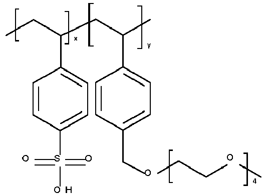 | Polyanion-01 |
| 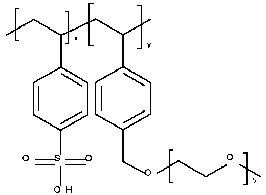 | Polyanion-02 |
| 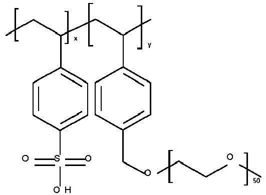 | Polyanion-03 |
| 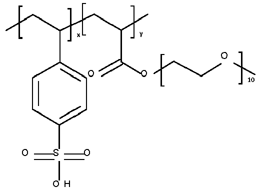 | Polyanion-04 |
| 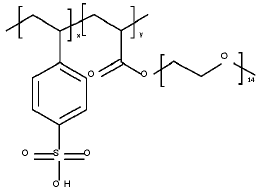 | Polyanion-05 |
| 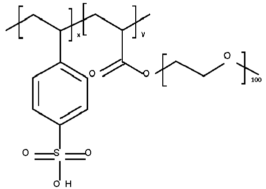 | Polyanion-06 |
| 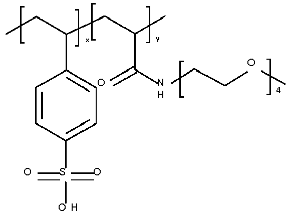 | Polyanion-07 |
| 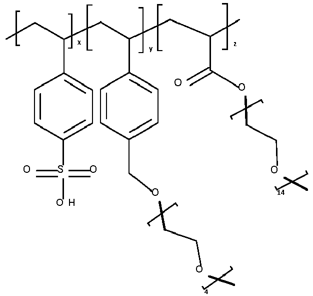 | Polyanion-08 |
| 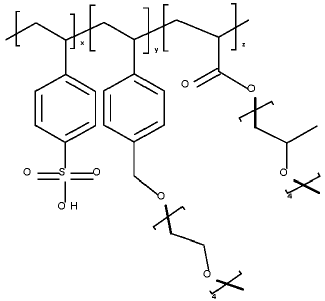 | Polyanion-09 |
| 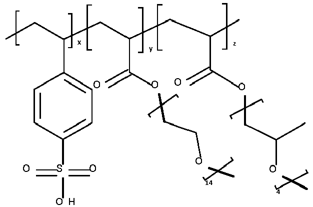 | Polyanion-10 |

x + y + z is 1. y is preferably from 0.001 to 0.5, more preferably from 0.01 to 0.25, most preferably from 0.02 to 0.1. z is preferably lower than y.

The polyanion is preferably prepared by a radical polymerization. More preferably, the polyanion is prepared by a free radical polymerization of styrene sulfonic acid and at least one monomer according to Formula II or a free radical polymerization of styrene sulfonic acid and a precursor that can be modified post polymerization to obtain a polyanion described in Table 2. The free radical polymerization is preferably carried out in an aqueous reaction medium.

In addition to the polyanion described above, the polythiophene/polyanion composition may comprise one or more other polyanions. For example, the polyanion described above may be combined with polystyrene sulphonic acid (PSS).

### Preparation of the polythiophene/polyanion composition

The polythiophene is preferably prepared by oxidative polymerization of the thiophene monomers described above. More preferably, the conductive polymer is prepared by oxidative polymerization of the thiophene monomers described above in an aqueous reaction medium. The oxidative polymerization is preferably carried out in the presence of the polyanion described above.

The concentration of the thiophene monomer in the aqueous medium is preferably from 0.1 to 25 wt%, preferably from 0.5 to 10 wt%, all relative to the total weight of the aqueous reaction medium.

Suitable oxidizing agents are iron(III) salts, such as FeCl₃, and iron(III) salts of aromatic and aliphatic sulfonic acids; H₂O₂; K₂Cr₂O₇; KMnO₄, alkali metal perborates; alkali metal or ammonium persulfates; and mixtures thereof.

Further suitable oxidants are described, for example, in Handbook of Conducting Polymers (Ed. Skotheim, T. A., Marcel Dekker: New York, 1986, Vol. 1, pages 46-57).

Particularly preferred oxidizing agents are salts of a peroxydisulfate, in particular K₂S₂O₈ and Na₂S₂O₈; iron(III) salts, in particular iron(III) chloride and iron(III) sulphate; or a combination thereof.

A mixture of salts of a peroxydisulfate and at least one further compound that catalyzes the cleavage of the peroxydisulfate, such as an iron(III) salt, is particularly preferred.

According to a particularly preferred embodiment the oxidizing agent is a mixture of Fe₂(SO₄)₃ and Na₂S₂O₈.

There are different ways for preparing the aqueous reaction medium. The thiophene monomer can be dissolved or dispersed in the aqueous reaction medium followed by the addition of the oxidizing agent(s), which can also be dissolved or dispersed in an aqueous phase, or the oxidizing agent(s) is/are first dissolved or dispersed in the aqueous reaction medium, followed by the addition of the thiophene monomer, which can also be dissolved or dispersed in an aqueous phase.

If more than one oxidizing agent is used, for example a mixture of Fe₂(SO₄)₃ and Na₂S₂O₈, it is furthermore possible to first mix one of these components with the thiophene monomer in the aqueous reaction medium followed by the addition of the second oxidizing agent.

The oxidative polymerization is preferably carried out under an inert atmosphere as disclosed in EP-A 11453877 (AGFA-GEVAERT NV). The oxygen content of the reaction medium when the oxidizing agent, for example a salt of peroxydisulfate, is added to it is preferably less than 3 mg per liter, more preferably less than 1.5 mg/liter, most preferably less than 0.5 mg/liter.

The concentration of oxygen in the reaction medium can be regulated by any means, such as freeze-thaw techniques, prolonged bubbling of an inert gas such as Argon, Nitrogen or Helium through the reaction medium, consumption of oxygen in a sacrificial reaction under an inert gas blanket. Preferably, an inert gas is bubbled through the reaction medium until the polymerization is completed thereby maintaining the oxygen concentration below 3 mg/l.

The oxidative polymerization is preferably carried out at low pH, as disclosed in EP-A 1384739. The pH is preferably 1.5 or less, more preferably 1.00 or less. To adjust the pH, an acid may be used, preferably selected from the group of water-soluble inorganic acids and water-soluble organic acids. Examples of inorganic acids are hydrochloric acid, sulfuric acid, nitric acid and phosphoric acid. Examples of organic acids include p-toluenesulfonic acid, benzenesulfonic acid, methanesulfonic acid and trifluoromethanesulfonic acid.

The temperature of the reaction mixture is preferably between 0 to 100°C, more preferably between 0 to 50°C, most preferably between 5 and 30°C.

The amount of thiophene monomers and polyanions in the reaction mixture is typically optimized to obtain stable polythiophene/polyanion dispersions of which the solid content is preferably from 0.05 to 25 wt%, more preferably from 0.1 to 10 wt%, most preferably from 0.8 to 2 wt%, all relative to the total weight of the dispersion.

After the polymerization reaction is completed the liquid composition may be further purified, for example by means of filtration, in particular by means of ultrafiltration, and/or by a treatment with ion exchanger, in particular by a treatment with an anion exchanger and a cation exchanger.

Various homogenization techniques may be used during the preparation of the polythiophene/polyanion complex. The homogenizing techniques may be selected from:
- ultrasonic homogenization techniques;
- pressure homogenization techniques; and
- mechanical homogenization techniques.

Preferred mechanical homogenizers are rotor-stator homogenizers and blade type homogenizers. Another mechanical homogenization technique may be the use of a spinning disk reactor.

Preferred high-pressure homogenizers, such as for example a Gaulin homogenizer or an Ariete homogenizer, force the dispersion through a very narrow channel or orifice under pressure. Another preferred high-pressure homogenizer is a microfluidizer.

Two or more homogenizers may be used in combination, preferably in a consecutive way.

The homogenization techniques may be used before, during and after the polymerization reaction.

### Surface-active compounds

The surface-active compounds may be:
- anionic surfactants such as alkylbenzenesulphonic acids and salts, paraffin sulphonates, alcohol sulphonates, ether sulphonates, sulphosuccinates, phosphate esters, alkyl ether carboxylic acids or carboxylates;
- cationic surfactants such as quaternary alkylammonium salts;
- nonionic surfactants such as linear alcohol ethoxylates, oxo alcohol ethoxylates, alkylphenol ethoxylates or alkyl polyglucosides; and
- zwitterionic surfactants such as compounds comprising both a carboxylic acid group and a quaternary ammonium group, for example lauryl-*N*,*N* -(dimethyl-ammonio)-butyrate and lauryl-*N*,*N*-(dimethyl)-glycinebetaine; compounds comprising both a sulfuric acid group and quaternary ammonium group, for example 3-[(3-cholamido-propyl)dimethylammonio]-1-propane-sulfonate, 3-(4-*tert*-butyl-1-pyridinio)-1-propanesulfonate, 3-(1-pyridinio)-1-propanesulfonate and 3-(benzyl-dimethyl-ammonio)propanesulfonate; compounds comprising both a phosphoric acid group and a quaternary ammonium group, for example with hexadecyl phosphocholine; compounds comprising a quaternary ammonium group with an attached hydroxy group, for example lauryldimethylamine *N*-oxide; and phospholipids, which consist of a quaternary ammonium head coupled *via* a phosphate group and glycerol to two hydrophobic fatty acids.

Particularly preferred surfactants are the commercially available surfactants available under the trademarks Dynol^{®} and Zonyl^{®}.

### Adhesion promoters

Preferred adhesion promoters are organofunctional silanes or hydrolysates thereof such as 3-glycidoxypropyltrialkoxysilane, 3-amino-propyl-triethoxysilane, 3-mercaptopropyltrimethoxysilane, 3-methacryloxypropyl-trimethoxysilane, vinyltrimethoxysilane or octyltriethoxysilane.

### Crosslinking agents

Preferred crosslinking agents are melamine compounds, blocked isocyanates, functional silanes such as tetraethoxysilane, alkoxysilane hydrolysates, such as tetraethoxysilane, epoxysilanes such as 3-glycidoxy-propyltrialkoxysilane.

### Binders

Preferred binders are polyurethanes, polyacrylates or polyolefins.

### Conductivity increasing compounds

Preferred conductivity increasing compounds are:
- compounds containing ether groups such as e.g. tetrahydrofuran;
- compounds containing lactone groups such as γ-butyrolactone or γ-valero-lactone;
- compounds containing amide or lactam groups such as caprolactam, N-methylcaprolactam, N,N-dimethylacetamide, N-methylacetamide, formamide, N,N-dimethylformamide (DMF), N-methyl-formamide, N-methyl-formanilide, N-methyl-2-pyrrolidone (NMP), N-octyl-pyrrolidone, 2-pyrrolidone, N-butyl-pyrrolidone, and N-hydroxyethyl-pyrrolidon;
- sulphones and sulphoxides such as sulpholane (tetramethylene sulphone) or dimethylsulphoxide (DMSO);
- sugar or sugar derivatives such as arabinose, saccharose, glucose, fructose or lactose;
- di- or polyalcohols such as sorbitol, xylitol, mannitol, mannose, galactose, sorbose, gluconic acid or ethylene glycol, di- or tri(ethylene glycol), 1,1,1-trimethylol-propane, 1,3-propanediol, 1-,2-propane-diol, 1,5-pentanediol, 1,2,3-propanetriol, 1,2,4-butanetriol or 1,2,6-hexanetriol, aromatic di- or polyalcohols such as resorcinol.

Particularly preferred conductivity increasing compounds are selected from the groups consisting of N-methyl-pyrrolidinone, N-butyl-pyrrolidone, N-hydroxyethyl-pyrrolidone, DMSO, ethylene glycol and diethylene glycol.

### Stability improving compounds

Preferred stability improving compounds are gallic acid derivatives.

### Polymer capacitor

A polymer capacitor, also referred to as a polymer electrolyte capacitor, is an electrolytic capacitor comprising a solid conductive polymer electrolyte.

Electrolytic capacitors use a chemical feature of some special metals, often referred to as so-called valve metals, that by anodic oxidation form an insulating oxide layer. By applying a positive voltage to the anode material in an electrolytic bath, an oxide barrier layer with a thickness corresponding to the applied voltage may be formed. This oxide layer acts as the dielectric in the electrolytic capacitor. To increase the capacitors capacitance the anode surface is roughened before anodization, resulting in roughened oxide layer surface.

To complete the capacitor, a counter electrode has to match the rough insulating oxide surface. This is accomplished by the electrolyte, which acts as the cathode of the electrolytic capacitor. In a polymer electrolyte capacitor, this counter electrode consists of one or more layers of a conductive polymer, for example a polythiophene conductive polymer. In the capacitor according to the present invention the cathode includes the polythiophene/polyanion complex described above.

The main difference between the polymer capacitors is the anode material and its oxide used as the dielectric:
- Polymer tantalum electrolytic capacitors use high purity sintered tantalum powder as an anode with tantalum pentoxide (Ta₂O₅) as a dielectric; and
- Polymer aluminum electrolytic capacitors use a high purity and electrochemically etched (roughened) aluminum foil as an anode with aluminum oxide (Al₂O₃) as the dielectric.

Preferred capacitors according to the present invention are polymer aluminum capacitors.

The porous metal layer (anode) covered with its oxide layer (dielectric) is hereinafter referred to herein as a porous anode body.

The polymer capacitor according to the present invention includes the polythiophene/polyanion composition described above.

The polymer capacitor according to the present invention is preferably prepared by a method comprising the step of introducing the polythiophene/polyanion composition described above into at least part of a porous anode body.

The polythiophene/polyanion composition may be introduced into the porous anode body by any known process such as impregnation, dipping, pouring, dripping on, spraying, misting on, knife coating, brushing or printing, for example ink-jet, screen or tampon printing.

Preferably, the polythiophene/polyanion composition is introduced into at least part of the porous anode body by dipping the porous anode body into the polythiophene/polyanion composition and thus impregnating it with the polythiophene/polyanion composition.

The dipping into or the impregnation with the polythiophene/polyanion composition is preferably carried out for a period of from 1 second to 120 minutes, more preferably of from 5 seconds to 60 minutes, most preferably in a range of from 10 seconds to 15 minutes. The introduction of the polythiophene/polyanion composition into the anode body can be facilitated, for example, by increased or reduced pressure, vibration, ultrasound or heat.

After the porous anode bodies have been impregnated with polythiophene/polyanion composition, solvents contained in the composition are preferably, at least partially, removed to obtain a solid electrolyte which completely or partly covers the dielectric thereby forming a capacitor body. The coverage of the dielectric by the solid electrolyte is preferably at least 10%, more preferably at least 25%, most preferably at least 50%. The coverage may be determined as is described in DE-A-10 2005 043 828.

The solvents are preferably removed by removing the electrode body from the composition and drying it. The drying step is preferably carried out at a temperature of from 20 °C to 260 °C, more preferably of from 50 °C to 220 °C, most preferably of from 80 °C to 200 °C.

The dipping and the drying step may be repeated once or several times to adapt the thickness of the solid electrolyte layer deposited on the dielectric or the degree of filling of the electrolyte in the electrode body to the particular requirements.

It may be advantageous to use both so-called self-doped and foreign-doped polythiophenes for the formation of the polymer cathode layer. A polythiophene typically has a positive charge located on the main chain of the polymer. The positive charge is preferably, at least partially, compensated by an anion. When the anion is covalently bound to the polymer, the polymer is then often referred to as a self-doped polymer or an intrinsically conductive polymer. The monomers used to make such self-doped polymers, i.e. comprising an anionic group, are also referred to as self-doped monomers.

When the anion is a separate compound, as is the case in the polythiophene/ polyanion composition described above, the polymer is then typically referred to as a foreign-doped polymer or an extrinsically conductive polymer. Anions added as separate compounds are preferably polyanions.

Both types of polythiophene polymers may be combined in a single composition and introduced as described above. However, it is preferred that both types of polythiophenes are introduced in the capacitor using different compositions each comprising a self-doped or a foreign- doped polythiophene. Preferably, first a self-doped polythiophene is introduced into the porous anode body followed by the introduction of the foreign-doped polythiophene. Using both self-doped and foreign-doped polythiophenes is disclosed in for example WO2014/048562 (Heraeus) and US2016/0351338 (AVX).

After the capacitor bodies have been produced in this manner, they can be further modified by the method and manner known to the person skilled in the art. In the case of a tantalum electrolytic capacitor, the capacitor bodies can be covered, for example, with a polymeric outer layer, as is described in DE-A-10 2004 022674 or DE-A-10 2009 007 594, and/or a graphite layer and a silver layer, as is known from DE-A-10 2005 043 828, while in the case of an aluminum wound capacitor, in accordance with the teaching of US749879, the capacitor body is incorporated into an aluminum beaker, provided with a sealing glass and firmly closed mechanically by crimping. The capacitor can then be freed from defects in the dielectric in a known manner by ageing.

### Lithium Ion battery

Secondary lithium ion batteries operate by reversibly passing lithium ions between the negative electrode (anode) and the positive electrode (cathode). A separator and an electrolyte are disposed between the negative and positive electrodes. The electrolyte is suitable for conducting lithium ions and may be in solid or liquid form. Lithium ions move from the cathode to the anode during charging of the battery, and in the opposite direction when discharging the battery. Both electrodes include an electroactive material.

The cathode or positive electrode for a Li-ion battery according to the present invention includes a polythiophene/polyanion composition described above.

The cathode or positive electrode for a Li-ion battery according to the present invention is preferably prepared by applying a cathode active composition on a current collector, preferably an Aluminum foil. The cathode active composition includes:
- a positive electroactive material,
- the polythiophene/polyanion composition described above, and
- a solvent.

The cathode active composition may further comprise a binder, other conductive agents, dispersant, etc.

The applied cathode active composition is then preferably dried to, at least partially, remove the solvent. The drying step is preferably carried out at a temperature from 20 °C to 260 °C, more preferably from 50 °C to 220 °C, most preferably from 80 °C to 200 °C.

The positive electroactive material and the polythiophene/polyanion complex may form core-shell particles wherein the core includes the electroactive material and the shell includes the polythiophene/polyanion complex, the optional binder and the optional conductive agents.

The positive electroactive material of the positive electrode or cathode include, but is not limited, to a layered metal oxide.

The metal oxide may be selected from:
- lithium cobalt oxide (LiCoO₂) or lithium nickel oxide (LiNiO2), or a compound substituted with one or more transition metals;
- lithium manganese oxides represented by formula Li₁₊ₓMn₂₋ₓO4 (0 ≤ x ≤ 0.33) such as LiMnOs, LiMn₂O₃, LiMnO2;
- a lithium copper oxide (Li₂CuO₂) ;
- LiFe₃O₄;
- vanadium oxides such as LiV₃O₈, V₂O₅, Cu₂V₂O₇;
- Ni site-type lithium nickel oxides represented by formula LiNi₁₋ₓMₓO₂ (M = Co, Mn, Al, Cu, Fe, Mg, B or Ga, and 0.01 ≤ x ≤ 0.3);
- lithium manganese composite oxides represented by formula LiMn₂₋ₓMₓO₂ (M = Co, Ni, Fe, Cr, Zn or Ta, and 0.01 ≤ x ≤ 0.1) or Li₂Mn₃MO₈ (M = Fe, Co, Ni, Cu or Zn);
- lithium manganese composite oxides having a spinel structure represented by LiNiₓMn₂₋ₓO4; LiMn₂O₄ in which a part of Li in the formula is substituted with an alkaline earth metal ion,
- Lithium iron phosphate (LiFePO₄).

A preferred metal oxide is LiNiₓMn_{y}Co_{1-x-y}O₂, also referred to as NMC. A particularly preferred metal oxide is LiNiₓMn_{y}Co_{1-x-y}O₂ with a high Ni content (Ni ≥ 0.8).

The discharge capacity of NMC materials is generally determined by the Ni content, as Ni is the major redox species (Ni²⁺/Ni⁴⁺).Thus higher Ni content cathode active materials guarantee a high specific capacity. As a result, a high energy density can be obtained. However, NMC cathode materials with higher Ni content have shown poor rate capability, poor cycling life and poor thermal stability, due to structural instabilities.

The amount of electroactive material is preferably from 75 to 99 wt%, more preferably form 80 to 95 wt%, most preferably from 85 to 95 wt%, all relative to the total weight of the cathode active composition.

The solvent is preferably N-methylpyrrolidone (NMP) or water, more preferably water.

The binder is preferably selected from the group consisting of styrene-butadiene rubber (SBR), carboxymethyl cellulose (CMC), acrylonitrile copolymer, polyacrylic acid (PAA), polyacrylonitrile (PAN), polyacrylic latex such as LA132, LA133 or LA138, a salt of alginic acid, polyvinylidene fluoride (PVDF), poly(vinylidene fluoride)hexafluoripropene (PVDF-HFP), polytetrafluorethylene (PTFE), polystyrene, poly(vinyl alcohol) (PVA), polyvinyl acetate, polyisoprene, polyaniline, polyethylene, polymide, polyurethane, polyvinyl butyral (PVB), polyvinyl pyrrolidone (PVP), gelatin, chitosan, starch, agar-agar, xanthan gum, gum arabic, gellan gum, guar gum, gum karaya, tara gum, gum tragacanth, casein, amylose, pectin, carrageenans, and combinations thereof. The type of binder used is typically dependent on the solvent used. For NMP a preferred binder is polyvinylidene fluoride (PVDF), for water the binder is preferably CMC, sodium alginate or PAA.

The binder amount is preferably from 0.01 to 10 wt %, more preferably from 0.1 to 7.5 wt%, most preferably from 1 to 5 wt%, all relative to the total weight of the cathode active composition.

The current collectors used are not particularly limited as long as they have a high conductivity without causing a chemical change in the battery. The current collector may be, for example, copper, stainless steel, aluminum, nickel, titanium, baked carbon, those that the surface of copper or stainless steel is surface-treated with carbon, nickel, titanium, silver, or the like, an aluminum-cadmium alloy, and the like. The current collector may have a thickness of 3 µm to 500 µm.

The adhesion of the electrode active material on the current collector may be strengthened by forming fine irregularities on the surface of the current collector. The current collector may be used in various forms of, for example, a film, a sheet, a foil, a net, a porous body, a foam, a non-woven body, and the like.

A particularly preferred positive electrode current collector is an aluminum foil while a particularly preferred negative electrode current collector is a copper foil.

The separator is preferably a microporous polymer film having pores with a size of several nm to several mm, which separates the negative electrode and the positive electrode and provide passages for lithium ions. The separator can be used without any particular limitation as long as it is usually used as a separator in a lithium secondary battery, and in particular, it is preferable that the separator has low resistance to ion movement of the electrolyte as well as excellent electrolyte moisture content capability.

The separator may be, for example, a porous polymer film made of a polyolefin-based polymer such as ethylene homopolymer, propylene homopolymer, ethylene/butene copolymer, ethylene/hexene copolymer, and ethylene/methacrylate copolymer, or a laminated structure of two layers thereof, and the like, or may be a conventional porous nonwoven fabric made of high-melting glass fiber, polyethylene terephthalate fiber, and the like, and in another example, a coating separator comprising a ceramic component or a polymer material may also be used to secure heat resistance or mechanical strength.

The thickness of the separator may be, for example, from 1 µm to 100 µm, more preferably from 5 to 75 µm, most preferably from 10 to 50 µm.

The negative electrode of the lithium ion battery may comprise a negative current collector and a negative electrode active material formed on the negative electrode current collector described above.

The anode is typically made up of graphite, coated on a current collector, preferably a copper foil. Silicon Carbon (Si-C) composites are also used to prepare anode materials. Other type of anode materials are Silicon and Li-metal.

Liquid electrolytes in lithium-ion batteries consist of lithium salts, such
as LiPF₆, LiBF₄ or LiClO₄ in an organic solvent, such as ethylene carbonate, dimethyl carbonate, ethyl methyl carbonate and diethyl carbonate. A liquid electrolyte acts as a conductive pathway for the movement of cations passing from the negative to the positive electrodes during discharge.

Recent advances in battery technology involve using a solid as the electrolyte material. The most promising of these are ceramics. Solid ceramic electrolytes are mostly lithium metal oxides, which allow lithium-ion transport through the solid more readily due to the intrinsic lithium. The main benefit of solid electrolytes is that there is no risk of leaks, which is a serious safety issue for batteries with liquid electrolytes.

### Other applications

The polythiophene/polyanion composition according to the present invention is preferably used in polymer capacitors and Li-ion batteries as described above. However, the polythiophene/polyanion composition may be used in any electronic device, such as is selected from photoconductive cells, photoresistors, photoswitches, phototransistors, phototubes, IR detectors, photovoltaic device, solar cells, coating materials for memory storage devices, field effect resistance devices, anti-static films, biosensors, electrochromic devices, solid electrolyte capacitors, hybrid capacitors, super capacitors, energy storage devices, batteries and electromagnetic shielding.

### EXAMPLES

### Materials

All materials used in the following examples were readily available from standard sources such as ALDRICH CHEMICAL Co. (Belgium) and ACROS (Belgium) unless otherwise specified. The water used was deionized water.

**Monomer-13** is a polyethylene glycol monomethyl ether acrylate commercially available from TCI Europe (Belgium).

**Monomer-16** is a polyethylene glycol monomethyl ether acrylate commercially available from TCI Europe (Belgium).

**4-vinylbenzenesulfonic acid,** sodium salt commercially available from TCI Europe (Belgium).

EDOT is 3,4-ethylenedioxythiophene commercially available from Heraeus.

**PSS-1** is an aqueous solution of polystyrenesulfonic acid having a Mw of 300 kDa prepared according to a method disclosed in Houben-Weyl, Methoden der organischen Chemie, Vol. E 20, Makromolekulaire Stoffe, Teil 2 (1987), page 1141.

**Lewatit ^{®} MonoPlus M600** is a basic, gelular anion exchange resin commercially available from Lanxess AG.

**Lewatit ^{®} MonoPlus S 108 H** is an acidic, gelular cation exchange resin commercially available from Lanxess AG.

### Methods

### Molecular weight measurement

The molecular weight of the polymers was determined by gel permeation chromatography (GPC) on a Waters e2695 alliance with a 2998A PDA detector, calibrated with polystyrene sulfonate standards. The molecular weight distribution (polydispersity Ð) is calculated with Waters Empower 3.

### Nuclear magnetic resonance measurement

The molar amount of monomer according to this invention in the copolymer polyanion was determined by nuclear magnetic resonance (NMR) on a JEOL ECZ400R with Royal probe in D₂O (at = 2.04" - d1 = 10" - nt = 64 - 25 gr C - pw45).

### Viscosity measurement

The viscosity was measured with a glass capillary viscometer.

### Surface resistance measurement

The surface resistance SER was measured at room temperature using a two point probe method.

### Capacitance measurement

The capacitance of a capacitor was measured at 120 Hz at room temperature with a potentiostat.

### ESR measurement

The equivalent series resistance (ESR) was measured at 100 kHz at room temperature with a potentiostat.

### Example 1: 4-Vinylbenzyltetraethyleneglycol monomethylether (Monomer-01)

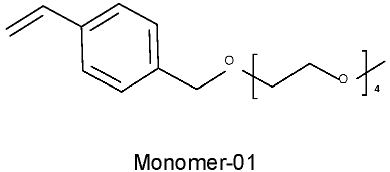

Tetraethyleneglycol monomethylether (12.2 g) was dissolved in 28 mL of THF and stirred. Then, a 60 wt% dispersion of sodium hydride in mineral oil (3.4 g) was added in portions. After 1 hour of stirring, a solution of 4-vinylbenzylchloride (6.8 g) in THF (12 mL) was added dropwise. The reaction mixture was further stirred for 20 hours while refluxing. Afterwards, the mixture was allowed to cool down to room temperature and slowly added to 150 mL of 0.3 M HCl at 0 °C. 200 mL of dichloromethane was added and the organic phase was extracted. The extraction was repeated with dichloromethane, a concentrated NaHCO₃ solution in water (150 mL) and brine (150 mL). The organic phase was isolated, dried on magnesium sulfate and the solvent was evaporated. Monomer-01 was obtained as a yellow oil (12.0 g, 92.5%).

### Example 2: 4-Vinylbenzylpentaethyleneglycol monomethylether (Monomer-02)

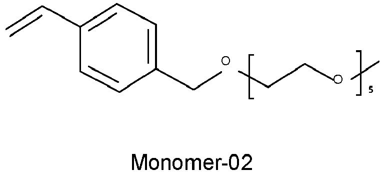

Pentaethyleneglycol monomethylether (4.6 g) was dissolved in 12 mL of THF and stirred. Then, a 60 wt% dispersion of sodium hydride in mineral oil (1.0 g) was added in portions. After 1 hour of stirring, a solution of 4-vinylbenzylchloride (2.0 g) in THF (3 mL) was added dropwise. The reaction mixture was further stirred for 20 hours while refluxing. Afterwards, the mixture was allowed to cool down to room temperature and slowly added to 100 mL of water at 0 °C. 100 mL of ethyl acetate was added and the organic phase was extracted. The extraction was repeated with ethyl acetate and brine (100 mL). The organic phase was isolated, dried on magnesium sulfate and the solvent was evaporated. Monomer-02 was obtained as a yellow oil (4.1 g, 92.7%).

### Example 3: Preparation of Polyanion-01(a) and Polyanion-01(b)

### Polyanion-01(a)

21.66 g of 4-vinylbenzenesulfonic acid, sodium salt and 0.70 g of Monomer-01 from Example1 are dissolved in 128.54 mL of water. A 2 wt% initiator solution of 2,2'-azobis[2-methyl-N-(2-hydroxyethyl)propionamide] was prepared and degassed with nitrogen for 1 hour. 51.46 g of this solution was added. The reaction mixture was then heated and refluxed for 20 hours. Afterwards, the reaction mixture was cooled to room temperature. The reaction mixture was treated with ion exchanger (120 g Lewatit ^{®} MonoPlus S 108 H, filtered and washed 2x 34 mL water, repeated). Afterwards, the solution dialyzed with dialysis tubes with a cutoff point of 1000 Da under a water flow. The solution was concentrated in vacuo. This procedure yielded a clear to slightly yellow solution of Polyanion-01(a) in water.

### Polvanion-01(b)

A polymerization was carried out in the same manner as described for Polyanion-01(a) except for using 20.61 g of 4-vinylbenzenesulfonic acid, sodium salt, 50.50 g of initiator solution, 129.5 mL of water and 1.71 g of Monomer-01.

### Example 4: Preparation of Polyanion-02(a) to Polyanion-02(c)

### Polyanion-02(a)

A polymerization was carried out in the same manner as described for Polyanion-01(a) except for using 10.78 g of 4-vinylbenzenesulfonic acid, sodium salt, 25.61 g of initiator solution, 64.39 mL of water and 0.39 g of Monomer-02 of Example 2.

### Polvanion-02(b)

A polymerization was carried out in the same manner as described for Polyanion-02(a) except for using 10.19 g of 4-vinylbenzenesulfonic acid, sodium salt, 24.96 g of initiator solution, 65.04 mL of water and 0.96 g of Monomer-02.

### Polyanion-02(c)

A polymerization was carried out in the same manner as described for Polyanion-02(a) except for using 9.26 g of 4-vinylbenzenesulfonic acid, sodium salt, 23.95 g of initiator solution, 66.05 mL of water and 1.84 g of Monomer-02.

### Example 5: Preparation of Polyanion-04(a)

A polymerization was carried out in the same manner as described for Polyanion-01(a) except for using 10.65 g of 4-vinylbenzenesulfonic acid, sodium salt, 25.30 g of initiator solution, 64.70 mL of water and 0.51 g of Monomer-13.

### Example 6: Preparation of Polyanion-05(a)

A polymerization was carried out in the same manner as described for Polyanion-01(a) except for using 10.46 g of 4-vinylbenzenesulfonic acid, sodium salt, 24.84 g of initiator solution, 65.16 mL of water and 0.68 g of Monomer-16.

### Example 7

The Molecular Weight (Mw) in kDa of Polyanion-01(a) and Polyanion-01(b), Polyanion-02(a) to Polyanion-02(c), Polyanion-04(a) and Polyanion-05(a) was determined as described above. The results are shown in Table 3. The amount of monomers according of Formula II in mol% (mol% Monomer) and the amount of monomer according to NMR analysis are also shown in Table 3.

**Table 3**

| | **mol % Monomer** | **NMR mol%** | **Mw (kDa)** | **D** | **Conc. (wt%)** |
|---|---|---|---|---|---|
| **Polyanion-01(a)** | 2 | 2.0 | 160 | 10.3 | 5.45 |
| **Polyanion-01(b)** | 5 | 5.0 | 170 | 9.3 | 6.20 |
| **Polyanion-02(a)** | 2 | 1.9 | 138 | 6.79 | 6.00 |
| **Polyanion-02(b)** | 5 | 4.7 | 156 | 7.82 | 4.98 |
| **Polyanion-02(c)** | 10 | 10.0 | 159 | 6.97 | 4.11 |
| **Polyanion-04(a)** | 2 | 1.4 | 148 | 7.68 | 4.43 |
| **Polyanion-05(a)** | 2 | 1.0 | 178 | 4.69 | 11.29 |

### Example 8: Preparation of PEDOT/Polyanion-01(a)

58.98 g of Polyanion-01(a) obtained in Example 3, deionized water (239.74 mL) and nitric acid (2.28 g) are mixed in a reaction vessel. Iron(III) sulfate (0.12 g) and sodium persulfate (2.37 g) were added. The reaction mixture was stirred and cooled to 5 °C under nitrogen flow for 90 min. The oxygen level was below 30 ppb. 3,4-Ethylenedioxythiophene (EDOT) (1.29 g) was added to the reaction mixture and it was stirred for 20 hours at 5 °C under nitrogen. The reaction mixture was treated with high shear homogenization (Lab Gaulin, 2x 600 bar) followed by a treatment with ion exchanger (81.4 g Lewatit ^{®} MonoPlus M600 + 43.3 g Lewatit ^{®} MonoPlus S 108 H, filtered and washed 2x 60 mL water, repeated). The resulting viscous mixture was again treated with high shear homogenization (Lab Gaulin, 2x 600 bar). The dispersion was concentrated in vacuo. This procedure yielded a blue dispersion of PEDOT:Polyanion-01(a) in water (1.10 wt%).

### Example 9: Preparation of PEDOT/Polyanion-01(b)

A polymerization was carried out in the same manner as in Example 8 except for using 45.21 g of Polyanion-01(d) obtained in Example 3 and 253.51 mL of deionized water. This procedure yielded a blue dispersion of PEDOT:Polyanion-01(b) in water (1.23 wt%).

### Example 9: Preparation of PEDOT/Polyanion-02(a)

A polymerization was carried out in the same manner as in example 8 except for using 53.57 g of Polyanion-02(a) obtained in Example 4 and 245.14 mL of deionized water. Prior to the first high shear homogenization step, 200 mL of water was added to the reaction mixture. This procedure yielded a blue dispersion of PEDOT:Polyanion-02(a) in water (1.17 wt%).

### Example 10: Preparation of PEDOT/Polyanion-02(b)

A polymerization was carried out in the same manner as in Example 9except for using 64.54 g of Polyanion-02(b) obtained in Example 4 and 234.17 mL of deionized water. This procedure yielded a blue dispersion of PEDOT:Polyanion-02(b) in water (1.00 wt%).

### Example 11: Preparation of PEDOT/Polyanion-02(c)

A polymerization was carried out in the same manner as in Example 9 except for using 78.21 g of Polyanion-02(c) obtained in Example 4 and 220.51 mL of deionized water. This procedure yielded a blue dispersion of PEDOT:Polyanion-02(c) in water (1.02 wt%).

### Example 13: Preparation of PEDOT/Polyanion-04(a)

72.56 g of Polyanion-04(a) obtained in Example 5, deionized water (226.16 mL), nitric acid (2.28 g) are mixed in a reaction vessel. Iron(III) sulfate (0.12 g) and sodium persulfate (2.37 g) were added. The reaction mixture was stirred and cooled to 5 °C under nitrogen flow for 90 min. The oxygen level was below 30 ppb. 3,4-Ethylenedioxythiophene (EDOT) (1.29 g) was added to the reaction mixture and it was stirred for 20 hours at 5 °C under nitrogen. The reaction mixture was treated with ion exchanger (81.4 g Lewatit ^{®} MonoPlus M600 + 43.4 g Lewatit ^{®} MonoPlus S 108 H, filtered and washed 2x 60 mL water, repeated). The resulting viscous mixture was again treated with high shear homogenization (Lab Gaulin, 4x 600 bar). The dispersion was concentrated in vacuo. This procedure yielded a blue dispersion of PEDOT:Polyanion-04(a) in water (1.01 wt%).

### Example 14: Preparation of PEDOT/Polyanion-05(a)

A polymerization was carried out in the same manner as in Example except for using 28.47 g of Polyanion-05(a) obtained in Example 6 and 270.24 mL of deionized water. This procedure yielded a blue dispersion of PEDOT:Polyanion-05(a) in water (1.23 wt%).

### Comparative example 1: Polymerization of PEDOT:PSS

56.0 g of PSS-1, deionized water (243 mL), and nitric acid (2.28 g) were mixed in a reaction vessel. Iron(III) sulfate (0.12 g) and sodium persulfate (2.37 g) were added. The reaction mixture was stirred and cooled to 5°C under nitrogen flow. The oxygen level was below 30 ppb. EDOT (1.29 g) was added to the reaction mixture and stirred for 20 hours at 5°C. The reaction mixture was then treated with ion exchanger (81.4 g Lewatit ^{®} MonoPlus M600 + 43.4 g Lewatit^{®} MonoPlus S 108 H, filtered and washed 2x 30 mL water, repeated). The resulting viscous mixture was treated with high shear homogenization (Lab Gaulin, 4x 600 bar). After a concentration step in vacuo a 1.13 wt% blue dispersion of PEDOT/PSS-1(a) in water was obtained.

### Comparative example 2: Polymerization of PEDOT:PSS

A polymerization was carried out in the same manner as in Comparative example 1 to obtain a 1.07 wt% blue dispersion of PEDOT/PSS-1(b) in water.

### Comparative example 3: Polymerization of PEDOT:PSS

73.3 g of PSS-1, deionized water (325 mL), and sulfuric acid (6N solution in water, 7.03 g) were mixed in a reaction vessel at 30 °C. The reaction mixture was stirred under nitrogen flow. The oxygen level was below 30 ppb. EDOT (1.71 g) was added to the stirring mixture. A solution of iron(III) sulfate (0.078 g) and sulfuric acid (6N solution in water, 0.09 g) in 10 mL of deionized water was added. Then, sodium persulfate (3.44 g) added to the reaction mixture and stirred for 16 hours at 30 °C. The reaction mixture was then treated with ion exchanger (62 g Lewatit ^{®} MonoPlus M600 + 32 g Lewatit ^{®} MonoPlus S 108 H, filtered, repeated). The resulting viscous mixture was treated with high shear homogenization (Lab Gaulin, 4x 600 bar). After a concentration step in vacuo a 1.22 wt% blue dispersion of PEDOT/PSS-1(c) in water was obtained.

### Example 12

The viscosity of the dispersions and the SER of bar-coated films of these dispersions on PET are shown in Table 4.

**Table 4**

| | **Viscosity (mPa.s)** | **SER (Ω/□)** |
|---|---|---|
| **PEDOT/Polyanion-01(a)** | 97 | 55 |
| **PEDOT/Polyanion-01(b)** | 219 | 62 |
| **PEDOT/Polyanion-02(a)** | 53 | 62 |
| **PEDOT/Polyanion-02(b)** | 43 | 91 |
| **PEDOT/Polyanion-02(c)** | 50 | 110 |
| **PEDOT/Polyanion-04(a)** | 85 | 65 |
| **PEDOT/Polyanion-05(a)** | 89 | 56 |
| **PEDOT/PSS-1(a)** | 16 | 73 |
| **PEDOT/PSS-1(b)** | 15 | 71 |
| **PEDOT/PSS-1(c)** | 47 | 94 |

### Example 16: Preparation of capacitors

A chemically converted aluminum foil including an etching layer on a surface was prepared as a valve metal base. A dielectric layer was formed to cover the aluminum foil. The resulting chemically converted aluminum foil was used as an anode component. The rated voltage of the alumina layer was 90 V and the capacitance was 6.4 µF/cm². A 20 µm thick solder mask was printed on the AI foil with arrays of openings of 10 mm × 10 mm separated 10 mm from each other. The patterned foil was cut to 30 mm × 105 mm strips with 5 openings on them. The edge of the strips and the space between the openings were covered with tape.

The conductive polymer dispersion used in the manufacturing of the capacitors were treated with an ultrasound homogenization step to reduce the viscosity below 20 mPa.s and formulated with polyglycerin (0.257 parts for 1 part of PEDOT/Polyanion), diethyleneglycol (0.026 parts for 1 part of PEDOT/Polyanion), DYNOL^{™} 604 (0.001 for 1 part of PEDOT/Polyanion) and dimethylaminoethanol (up to a pH of 3.5±0.5).

The strip was dip-coated with the formulation of PEDOT/Polyanion-01(a) and dried at 150 °C for 5 minutes. The dip-coating and curing steps were repeated for several times. Then, carbon paste and silver paste were sequentially screen-printed on the PEDOT layer and cured. The tape was removed and the strips were cut to 5 capacitors. The capacitance and equivalent series resistance (ESR) were measured as described above.

Additional capacitors were prepared and measured as described above where PEDOT/Polyanion-01(b), PEDOT/Polyanion-02(a) to PEDOT/Polyanion-02(c), PEDOT/Polyanion-04(a), PEDOT/Polyanion-05(a), PEDOT/PSS-1(a), PEDOT/PSS-1(b) and PEDOT/PSS-1(c) were used as conductive polymer dispersions.

In Table 5 the results of the capacitor evaluation are summarized.

**Table 5**

| | **Capacitance (µF)** | **ESR (mΩ)** |
|---|---|---|
| **PEDOT/Polyanion-01(a)** | 7.4 | 37.0 |
| **PEDOT/Polyanion-01(b)** | 6.9 | 35.8 |
| **PEDOT/Polyanion-02(a)** | 7.1 | 43.7 |
| **PEDOT/Polyanion-02(b)** | 6.2 | 41.1 |
| **PEDOT/Polyanion-02(c)** | 6.2 | 41.7 |
| **PEDOT/Polyanion-04(a)** | 5.8 | 40.3 |
| **PEDOT/Polyanion-05(a)** | 5.8 | 42.5 |
| **PEDOT/PSS-1(a)** | 5.6 | 34.8 |
| **PEDOT/PSS-1(b)** | 5.6 | 37.9 |
| **PEDOT/PSS-1(c)** | 5.6 | 62.7 |

It is clear from the results in Table 5 that the dispersions wherein a polyanion according to the present invention is used, the capacitance of the capacitors can be increased and the ESR can be decreased while maintaining a comparable ESR.

## Claims

1. A polythiophene/polyanion composition comprising:
i) an oligo- or polythiophene obtained by the polymerization of a monomer according to Formula I, and 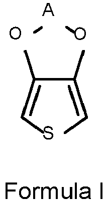 wherein
A represents a substituted or unsubstituted C1 to C5 alkylene bridge,
ii) a polyanion obtained by the polymerization of at least one monomer according to Formula II, 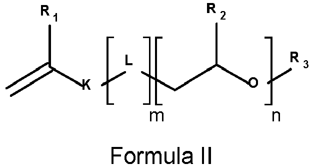 wherein
R₁ is selected from the group consisting of hydrogen, a substituted or unsubstituted alkyl group, a substituted or unsubstituted alkenyl group, a substituted or unsubstituted alkynyl group, a substituted or unsubstituted aralkyl group, a substituted or unsubstituted alkaryl group and a substituted or unsubstituted aryl or heteroaryl group; K is selected from the group consisting of a substituted or unsubstituted alkylene group, an ether group, an ester group, a substituted or unsubstituted amide group, a substituted or unsubstituted alkenylene group, a substituted or unsubstituted alkynylene group, a substituted or unsubstituted aralkylene group, a substituted or unsubstituted alkarylene group and a substituted or unsubstituted arylene or heteroarylene group;
L is selected from the group consisting of a substituted or unsubstituted alkylene group, a substituted or unsubstituted alkenylene group, an amine group, an ether group, a thioether group, a substituted or unsubstituted alkynylene group, a substituted or unsubstituted aralkylene group, a substituted or unsubstituted alkarylene group and a substituted or unsubstituted arylene or heteroarylene group;
R₂ and R₃ are independently selected from the group consisting of hydrogen and a substituted or unsubstituted alkyl group;
m represents 0 or 1;
n is an integer from 1 to 10 000;
and wherein the amount of monomer according to Formula II in the polyanion is from 0.1 to 20 mol %, relative to the total amount of monomers.

2. The polythiophene/polyanion composition according to claim 1 wherein m represents 0.

3. The polythiophene/polyanion composition according to claim 1 or 2 wherein R₁ represents a hydrogen or a methyl group.

4. The polythiophene/polyanion composition according any of the preceding claims wherein L represents an unsubstituted alkylene group.

5. The polythiophene/polyanion composition according any of the preceding claims wherein K represents a phenylene group.

6. The polythiophene/polyanion composition according any of the preceding claims wherein R₂ represents hydrogen and R₃ represents a methyl group.

7. The polythiophene/polyanion composition according any of the preceding claims wherein n is an integer from of 3 to 25.

8. The polythiophene/polyanion composition according any of the preceding claims wherein the polyanion is a copolymer obtained by the polymerization of at least one monomer according to Formula II and at least one other monomer.

9. The polythiophene/polyanion composition according to claim 8 wherein at least one other monomer is styrene sulphonic acid.

10. The polythiophene/polyanion composition according to claim 8 or 9 wherein the total amount of monomers according to Formula II is from 2 to 10 mol %, relative to the total amount of monomers of the polyanion.

11. The polythiophene/polyanion composition according any of the preceding claims wherein the oligo- or polythiophene is poly(3,4-ethylenedioxythiophene).

12. A method of preparing a polymer capacitor comprising a porous anode body including the step of introducing a polythiophene/polyanion composition as defined in any of the claims 1 to 11 into at least part of the anode body.

13. A method of preparing a cathode for a Li-ion battery including the step of applying a cathode active composition on a current collector, the cathode active composition comprising:
- a cathode electroactive material;
- a polythiophene/polyanion composition as defined in any of the claims 1 to 11; and
- a solvent.

14. The method according to claim 13 wherein the cathode electroactive material is a layered metal oxide and the solvent is water or N-methyl pyrrolidone (NMP).

15. The use of the polythiophene/polyanion composition as defined in any of the claims 1 to 11 for the preparation of a conductive layer in an electronic device.
